# DEMANDE DE BREVET EUROPEEN

(11) **EP 0 997 141 A1**
(43) Date de publication de la demande: **03.05.2000**
(21) Numéro de dépôt: 99402613.6
(22) Date de dépôt: 22.10.1999
(51) Int. Cl.: A61K 7/48, A61K 7/02

(54) **Composition cosmétique, contenant au moins une poly (cyclooléfine) amorphe ou un mélange de poly (cyclooléfines) amorphes, et leurs utilisations.**

(30) Priorité: 28.10.1998 FR 9813490
(71) Demandeur: L'OREAL, 75008 Paris (FR)
(72) Inventeur: Tournilhac, Florence, 75011 Paris (FR); Mondet, Jean, 93600 Aulnay-sous-Bois (FR)
(74) Mandataire: Casalonga, Axel

(57) **Abrégé**

La composition à application topique et en particulier cosmétique, comprend une phase grasse et une quantité efficace d'une poly(cyclooléfine) amorphe ou d'un mélange de poly(cyclooléfines) amorphes dissous ou dispersé dans une phase grasse liquide comprenant au moins une huile dont la structure chimique comporte au moins un noyau carboné.

Application : aux produits de maquillage et de protection solaire.

## Description

La présente invention a trait à une composition susceptible d'être appliquée sur les matières kératiniques et plus spécialement cosmétique contenant au moins une poly(cyclooléfine) amorphe ou un mélange de celles-ci, soluble ou dispersable dans une phase grasse liquide contenant au moins une huile dont la structure chimique comporte au moins un cycle carboné. Plus spécialement, l'invention se rapporte à une composition cosmétique, notamment à propriétés filmogènes et de bonne tenue, pour le soin et/ou le maquillage de la peau aussi bien du visage que du corps humain et du cuir chevelu, des muqueuses comme les lèvres et les paupières, ou encore des phanères comme les cils, les sourcils, les ongles et les cheveux.

Cette composition peut se présenter notamment sous forme épaissie pouvant aller jusqu'au produit coulé en stick ou en coupelle comme les rouges ou baumes à lèvres, les fonds de teint coulés, les produits anti-cernes, les fards à paupières ou à joues, sous forme de pâte ou de crème plus ou moins fluide comme les fonds de teint ou rouges à lèvres fluides, les eyes-liners, le maquillage du corps, les compositions de protection solaire ou de coloration de la peau, les mascaras, ou de poudres libres ou compactées.

Les produits de maquillage ou de soin de la peau ou des lèvres des êtres humains comme les fonds de teint ou les rouges à lèvres, contiennent généralement des phases grasses telles que des cires et des huiles, des pigments et/ou charges et, éventuellement des additifs comme des actifs cosmétiques ou dermatologiques. Elles peuvent aussi contenir des produits dits "pâteux", de consistance souple, permettant d'obtenir des pâtes, colorées ou non, à appliquer au pinceau.

Ces compositions, lorsqu'elles sont appliquées sur la peau ou les lèvres, présentent généralement l'inconvénient de transférer, c'est-à-dire de se déposer au moins en partie, en laissant des traces, sur certains supports avec lesquels elles peuvent être mises en contact, et notamment un verre, une tasse, une cigarette, un vêtement ou la peau. Il s'ensuit une persistance médiocre du film appliqué, nécessitant de renouveler régulièrement l'application de la composition, par exemple de fond de teint ou de rouge à lèvres. Par ailleurs, l'apparition de ces traces inacceptables, notamment sur les cols de chemisier, peut écarter certaines femmes de l'utilisation de ce type de maquillage. De plus, ces compositions sont souvent collantes et de résistance à l'eau et au sébum médiocre.

La Société PROCTER & GAMBLE a envisagé dans sa demande de brevet WO-A-96/36323 des compositions de mascara de type émulsion eau-dans-huile présentant une bonne tenue, une résistance à l'eau et ne laissant pas de traces. Ces compositions contiennent, entre autre, un polymère insoluble dans l'eau, appelé généralement un latex, associé à un tensio-actif du type alkyle ou alcoxydiméthicone copolyol, des huiles hydrocarbonées, des pigments et charges, ainsi que des cires.

Dans le document WO-A-97/01321, la Société REVLON a décrit une composition cosmétique "sans transfert", brillante, comprenant un polymère qui est un adhésif à température ambiante, un solvant volatil, une huile non volatile et une matière sèche particulaire. Les polymères adhésifs sont choisis parmi les polymères à squelette vinylique, méthacrylique ou acrylique et groupes pendants siloxane et fluorés, les polymères à squelette vinylique, méthacrylique ou acrylique et groupes pendants siloxanes, ainsi que les copolymères séquencés ou greffés vinyl-silicone.

En outre, les documents EP-A-497144 et FR-A-2 357 244 décrivent des compositions dites "sans transfert", contenant un polymère bloc styrène-éthylène-propylène associé à des cires, des huiles légères ou volatiles et des pigments.

Ces compositions présentent l'inconvénient d'être peu confortables, d'avoir des propriétés cosmétiques quelconques et d'être difficilement formulables. En particulier, lorsque les polymères sont liposolubles, ils conduisent à des films mous, collants, sans propriétés mécaniques véritables. Par ailleurs, les propriétés "sans transfert" de ces compositions sont très moyennes.

De plus, il existe, à ce jour, peu de polymères liposolubles épaississant d'huile en cosmétique, ayant de bonnes propriétés mécaniques et en particulier de bonnes propriétés filmogènes et qui conduisent à des compositions non collantes, ce qui limite quelque peu l'éventail des formulations.

Il subsiste donc le besoin d'une composition ne présentant pas les inconvénients ci-dessus, et en particulier ayant de bonnes propriétés de "sans transfert", notamment lors d'un frottement, une bonne tenue dans le temps, ayant de bonnes propriétés mécaniques, ne collant pas et/ou ne tiraillant pas la peau ou les lèvres sur lesquelles elle est appliquée et n'établissant pas d'inconfort par traitement d'un film solide.

La demanderesse a en particulier constaté, de façon tout à fait surprenante, que l'utilisation d'au moins une poly(cyclooléfine) amorphe, soluble ou dispersable dans une phase grasse liquide incluant au moins une huile dont la structure chimique comporte au moins un cycle carboné, en particulier dans une composition cosmétique, pouvait permettre d'obtenir un film, de très bonne tenue, et/ou résistant à l'eau, au sébum et au frottement et même à des solutions de tensio-actifs, tout en étant très agréable à l'application et à porter tout au long de la journée. Le film est notamment souple, flexible et non collant avec de bonnes propriétés mécaniques.

Dans la présente demande, on entend par poly(cyclooléfine) amorphe les homopolymères de cyclooléfine amorphes ainsi que les copolymères de cyclooléfine amorphes, tels que les copolymères oléfine/cyclooléfine amorphes.

Les poly(cyclooléfines) de l'invention sont amorphes et ont généralement une température de transition vitreuse (Tg) allant de -20°C à +300°C, et de préférence de -10°C à +150°C. Elles présentent notamment l'avantage d'être filmogènes et liposolubles tout en étant non collantes contrairement à la plupart des polymères filmogènes liposolubles utilisés en cosmétique. Ils permettent, en outre, un épaississement et/ou gélification de certains corps gras liquides. Or, il existe, à ce jour, peu de polymères épaississants de corps gras liquides, qui sont, en plus, filmogènes.

Les copolymères de cyclooléfines amorphes de l'invention, en particulier les copolymères oléfine/cyclooléfine, ont en général une teneur en cyclooléfine égale ou supérieure à 10 moles %, de préférence égale ou supérieure à 20 moles %. Ils présentent en particulier une température de transition vitreuse > -10°C.

La présente invention a donc pour objet une composition cosmétique susceptible d'être appliquée sur les matières kératiniques comprenant une phase grasse liquide, caractérisée par le fait qu'elle comprend une quantité efficace et en particulier au moins 1 % en poids, par rapport au poids total de la composition, d'au moins une poly(cyclooléfine) amorphe, la phase grasse liquide comprenant au moins une huile dont la structure chimique comporte au moins un cycle carboné.

Cette composition peut également contenir d'autres ingrédients compatibles avec la peau, les muqueuses et les phanères. En particulier, la phase grasse liquide peut contenir un ou plusieurs corps gras liquides, à température ambiante.

De préférence, cette composition contient en outre au moins une matière colorante.

Elle a également pour objet une composition cosmétique se présentant sous forme épaissie et notamment sous forme d'un produit coulé et comprenant au moins une phase grasse liquide, et éventuellement au moins une cire solide à température ambiante, caractérisée par le fait qu'elle comprend, en outre, une quantité efficace et mieux au moins 1 % en poids, par rapport au poids total de la composition, d'au moins une poly(cyclooléfine) amorphe, la phase grasse liquide comprenant au moins une huile dont la structure chimique comporte au moins un cycle carboné.

Comme précédemment, cette composition peut en outre contenir d'autres ingrédients compatibles avec la peau, les muqueuses et les phanères, et en particulier au moins une matière colorante.

La poly(cyclooléfine) amorphe ou le mélange de poly(cyclooléfines) amorphes, est présent en quantité efficace ou suffisante pour obtenir notamment un film ne transférant pas, en particulier au frottement, de bonne tenue, non collant, pouvant également être brillant et/ou résistant à l'eau et au sébum. Il peut, en outre, être présent en quantité suffisante pour épaissir et/ou gélifier une phase grasse liquide.

Les poly(cyclooléfines) amorphes utilisées dans les compositions de la présente demande peuvent être tout polymère ou copolymère de cyclooléfines amorphe ayant de préférence une Tg de -20°C à + 300°C, et mieux de -10°C à + 150°C, et encore mieux de 10°C à 150°C. De préférence, les poly(cyclooléfines) amorphes de l'invention ont une masse molaire (PM) variant de 1000 à 10⁶, de préférence de 10.000 à 500.000.

Ces poly(cyclooléfines) sont généralement des élastomères ou des polymères thermoplastiques résultant de la polymérisation ou copolymérisation d'au moins une cyclooléfine telle que, en particulier (et de façon non exhaustive) :
. cyclobutène, cyclohexène, cyclooctène,
. norbornène (c'est-à-dire bicyclo(2,2,1)heptène-2),
5-méthylnorbornène, 5-éthylnorbornène, 5,6-diméthylnorbornène, 5,5,6-triméthylnorbomène, 5-éthylidène-norbornène, 5-phényl-norbornène, 5-benzylnorbornène, 5-vinylnorbornène, 1,4,5,8-diméthano-1,2,3,4,4a,5,8a-octahydronaphtalène, dicyclopentadiène, ou de leurs mélanges.

On peut aussi utiliser des polymères comportant un cycle hydrocarboné dans leurs chaînes, issu de la polymérisation métallocène de certains diènes comme le 1,5-hexadiène conduisant à un motif "méthylène cyclopentène" dans le polymère.

De préférence, on utilise des copolymères plutôt que des homopolymères.

Parmi les copolymères de cyclooléfines, on préférera les copolymères (oléfines/cyclooléfines) de structure statistique, alternée, séquencée, multiséquencée, greffée, en étoile ou sous forme de tout autre architecture.

On préférera en particulier les copolymères entre au moins une des cyclooléfines citées précédemment et au moins une des oléfines choisies parmi l'éthylène, le propylène, le 1-butène, le 3-méthyl-1-butène, le 1-hexène, le 4-méthyl-1-pentène, le 1-octène, le 1-décène, le 1-éicosène, ou leurs mélanges.

Les poly(cyclooléfines) particulièrement recommandées de l'invention seront les copoly(éthylène/norbornène) et les terpolymères (éthylène/propylène/éthylidène-norbornène).

Ces polymères peuvent être synthétisés par tout procédé de polymérisation connu permettant de conserver la présence d'au moins un cycle hydrocarboné dans la chaîne principale ou latéral à cette chaîne.

Parmi les procédés de polymérisation les plus appropriés à ces homo ou copolymères de cyclooléfines, on citera plus particulièrement la catalyse métallocène (par exemple zirconocène/aluminoxane), telle que citée dans l'article de BERGSTRÖM C.H, J.Appl.Polym.Sci, 63(8) 1063-70 (1997) et dans l'article de MALMBERG A., J. Appl. of Polym.Sci. Vol 66, 35-44 (1997), et dans l'article de RISCHE T., Macromolecules, 31, 1871-74 (1998). Cette technique permet en particulier une bonne incorporation des cyclooléfines dans les copolymères avec les oléfines, sans ouvrir le ou les cycles portés par la ou les cyclooléfines. Comme autre technique de polymérisation adaptée à ces copolymères, on citera plus particulièrement la polymérisation par Métathèse, c'est-à-dire par ouverture de cycle de la cyclooléfine : on utilisera pour ce procédé des cyclooléfines possédant au départ au moins deux cycles, de façon qu'après polymérisation, la chaîne polymère comporte au moins un cycle (dans la chaîne ou en latéral). De telles cyclooléfines polycycliques seront en particulier le norbornène, le dicyclopentadiène, le 1,4,5,8-diméthano-1,2,3,4,4a,5,8a-octahydronaphthalène, etc...

L'invention couvre donc également les polymères obtenus à partir de cyclooléfines polycycliques, telles que norbornène, dicyclopentdiène, pentacyclopentadécène, polymérisées par Métathèse c'est-à-dire par ouverture de cycle. Pour ces monomères polycycliques, la polymérisation n'ouvre qu'un cycle et conduit toujours à un polymère possédant au moins un cycle répétitif dans la chaîne principale. A titre d'exemple, et à partir du norbornène on peut schématiser les polymérisations par voie métallocène et méthathèse de la manière suivante :

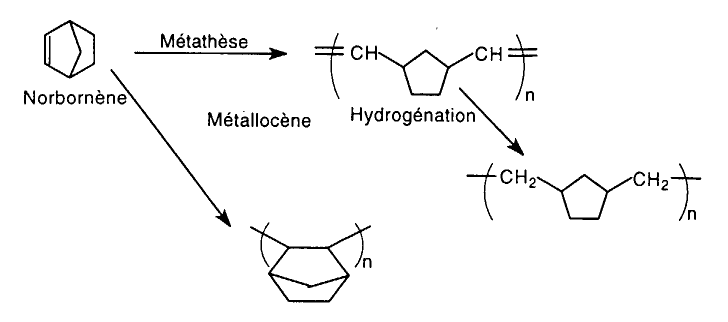

Les homo et copolycyclooléfines amorphes obtenues par Métathèse sont alors de préférence utilisées hydrogénées pour saturer les doubles liaisons de la chaîne principale. Cette saturation des doubles liaisons permet d'obtenir une meilleure stabilité des produits et des formulations les contenant.

Que ce soit par polymérisation Métallocène ou Métahèse, les procédés de polymérisation peuvent être de toute nature en solution, en phase gazeuse ou en milieu hétérogène.

D'autres méthodes de polymérisation plus traditionnelles telles que radicalaire ou Ziegler-Natta, peuvent être utilisées, à condition de pouvoir homo ou copolymériser efficacement la ou les cyclooléfines.

La polymérisation ou copolymérisation utilisant un de ces procédés, peut être effectuée en masse, en solution ou en dispersion.

Parmi les poly(cyclooléfines) amorphes utilisées, on peut citer en particulier les polynorbornènes et copolycycloalkylènes, copoly(éthylène/norbornène) commerciaux décrits dans l'article de BERGSTRÖM C.H., J. Appl. Polym. Sci. 63, (8) 1063-70, 1997 déjà cité. Plus particulièrement, les copolyoléfines réalisées par NIPPON ZEON sous le nom commercial de "ZEONEX®", par la société EXXON, par les sociétés HOECHST-CELANESE et TICONA sous le nom commercial de "TOPAS®". On citera également les terpolymères amorphes et commerciaux éthylène/propylène/éthylidène-norbornène tels que ceux réalisés par DUPONT/DOW sous le nom commercial NORDEL® IP (catégorie amorphe) .

Les copolymères oléfine/cyclooléfine préférés selon l'invention sont les poly(éthylène-co-norbornène) amorphes ayant en général une teneur en norbornène égale ou supérieure à 20 moles %, par exemple les poly(éthylène-co-norbornène) commercialisés par la société NIPPON ZEON sous le nom de ZEONEX® et par les sociétés HOECHST-CELANESE et TICONA sous le nom de TOPAS® 8007-S-04, et les poly(éthylène-co-cycloalkylène) amorphes, de préférence les poly(alkylène-co-cyclo(C₄-C₆)alkylène) tels que les poly(éthylène-co-cyclobutène) et les poly(éthylène-co-cyclohexène).

Parmi les copolymères d'oléfines/cyclooléfines amorphes préférés selon l'invention on peut encore citer plus généralement les copolymères obtenus par catalyse métallocène, en particulier en présence d'un couple zirconocène-aluminoxane.

Cette voie de synthèse permet en effet un très bon contrôle des poids moléculaires des copolymères et conduit à une faible polydispersité (Indice de polymolécularité ≤ 2). Elle permet un très bon contrôle de l'incorporation du comonomère dans les chaînes de polymères qui sont de composition chimique très voisine.

Pour plus de détails quant aux avantages de cette synthèse par catalyse métallocène, on pourra se reporter aux articles "Emerging Technologies In Polymer Science and Engineering" (Technologies émergentes dans la Science et l'Ingenierie des Polymères) M.P. ZAMORA et al. - Plactics Engineering/May 97, pages 75 à 79 ; J. Appl. Polym. Sci. 63 de CH. BERGSTRÖM, 1063-70 (1997) et J. Appl. Polym. Sci. 66 de A. MALMBERG, 35-44 (1997).

La poly(cyclooléfine) ou le mélange de poly(cyclooléfines) amorphe selon l'invention peut être choisi par l'homme du métier en fonction de ses propriétés et selon l'application ultérieure souhaitée pour la composition.

Ainsi, la poly(cyclooléfine) ou le mélange de poly(cyclooléfines) peut être filmifiable ou non. Cependant, l'obtention d'une composition de tenue prolongée dans le temps est plus spécialement due à l'utilisation d'une poly(cyclooléfine) ou d'un mélange filmifiable.

L'invention a également pour objet une composition telle que définie ci-dessus, comprenant une phase grasse notamment volatile et comprenant au moins un actif choisi parmi les actifs cosmétiques et les actifs dermatologiques.

Par "phase grasse volatile", on entend tout milieu non aqueux susceptible de s'évaporer de la peau ou des lèvres, en moins d'une heure.

L'invention a encore pour objet l'utilisation dans une composition non thérapeutique ou pour la fabrication d'une composition dermatologique susceptible d'être appliquée sur les matières kératiniques contenant au moins une phase grasse liquide comprenant au moins une huile dont la structure chimique comporte au moins un cycle carboné, d'une quantité efficace d'une poly(cyclooléfine) amorphe ou d'un mélange de poly(cyclooléfines) amorphes, soluble ou dispersable dans ladite phase grasse liquide, pour épaissir et/ou gélifier ladite phase grasse liquide.

Un autre objet de l'invention est l'utilisation dans ou pour la fabrication d'une composition épaissie et plus spécialement sous forme de produit coulé, susceptible d'être appliquée sur les matières kérainiques et comprenant au moins une phase grasse liquide comprenant au moins une huile dont la structure chimique comporte au moins un cycle carboné, et éventuellement au moins une cire, notamment solide à température ambiante, d'au moins une poly(cyclooléfine) amorphe soluble ou dispersable dans ladite phase grasse liquide, présente en une quantité efficace et notamment à au moins 1 % en poids, par rapport au poids total de la composition, pour obtenir un film de tenue prolongée et/ou sans transfert, de préférence brillant et/ou résistant à l'eau et/ou au sébum, ne collant pas et/ou confortable à porter.

Un autre objet de l'invention est l'utilisation dans ou pour la fabrication d'une composition, sans transfert, susceptible d'être appliquée sur les matières kératiniques d'au moins une poly(cyclooléfine) amorphe, filmifiable, soluble ou dispersable dans une phase grasse liquide, présente notamment à au moins 1 % en poids, par rapport au poids total de la composition, la phase grasse liquide comprenant au moins une huile dont la structure chimique comporte au moins un cycle carboné, pour diminuer et/ou supprimer le transfert du film de composition déposé sur les muqueuses et/ou la peau et/ou les phanères d'un être humain vers un support mis en contact avec le film.

Un autre objet de l'invention est l'utilisation dans ou pour la fabrication d'une composition susceptible d'être appliquée sur les matières kératiniques, comprenant une phase grasse liquide comprenant au moins une huile dont la structure chimique comporte au moins un cycle carboné, et au moins un ingrédient choisi parmi les actifs cosmétiques ou dermatologiques, les matières colorantes et leurs mélanges, d'au moins une poly(cyclooléfine) amorphe, soluble ou dispersable dans ladite phase grasse, présente notamment en une quantité efficace, et en particulier en une quantité d'au moins 1% en poids, par rapport au poids total de la composition, pour obtenir un film confortable, sans transfert au frottement et/ou de bonne tenue, de préférence brillant et/ou résistant à l'eau et/ou au sébum, ne collant pas et/ou confortable à porter.

L'invention a encore pour objet un procédé non thérapeutique et notamment cosmétique de soin ou de maquillage des lèvres, et/ou de la peau et/ou des phanères, consistant à appliquer sur respectivement les lèvres et/ou la peau et/ou les phanères, une composition non thérapeutique telle que définie précédemment.

L'invention a encore pour objet un procédé pour augmenter la tenue dans le temps et/ou la résistance à l'eau et/ou au sébum et/ou pour limiter le transfert d'une composition de maquillage ou de soin de la peau et/ou des lèvres et/ou des phanères sur un support différent de ladite peau et/ou desdites lèvres et/ou desdits phanères, contenant un phase grasse liquide et au moins un ingrédient choisi parmi les matières colorantes et les actifs cosmétiques et les actifs dermatologiques, consistant à introduire dans la phase grasse au moins une poly(cyclooléfine) amorphe soluble ou dispersable dans ladite phase grasse, présente en une quantité efficace et notamment à au moins 1% en poids, par rapport au poids total de la composition.

On a de plus constaté que les compositions selon l'invention présentent des qualités d'étalement et d'adhésion sur la peau, les semi-muqueuses, les muqueuses et les phanères, particulièrement intéressantes, ainsi qu'un toucher onctueux, non collant et agréable.

La phase grasse liquide dans laquelle est introduit le polymère ou copolymère amorphe, peut être constituée d'huiles cosmétiquement ou dermatologiquement acceptables, et de façon générale physiologiquement acceptables, notamment choisies parmi les huiles d'origine minérale, animale, végétale ou synthétique, carbonées, hydrocarbonées, fluorées et/ou siliconées, utilisées seules ou en mélange dans la mesure où elles forment un mélange homogène et stable, où elles sont compatibles avec l'utilisation envisagée et capables de solubiliser ou disperser le polymère ou copolymère de l'invention.

Par "phase grasse liquide", on entend tout milieu non aqueux liquide à température ambiante. Cependant, la phase grasse liquide comprend au moins une huile dont la structure chimique comporte au moins un cycle carboné et de préférence plusieurs cycles carbonés. Ces huiles peuvent être volatiles ou non. A titre d'exemple d'huiles à cycle carboné utilisables dans l'invention, on peut citer les (alkyl)cycloalcanes avec une chaîne alkyle linéaire ou ramifiée, saturée ou non ayant de 1 à 30 atomes de carbone et en particulier le cyclohexane, le dioctylcyclohexane, le benzène, le toluène, le 2,4-diméthyl-3-cyclohexène, le dipentène, le p-cymène, le naphtalène, l'anthracène, les dérivés de morpholine comme l'hydroxybenzomorpholine ; les esters comme le benzoate d'isostéaryle, les silicones phénylées comme les diphényldiméthicones. Ces huiles peuvent représenter de 1 à 99 % et mieux de 5 à 80 % en poids de la composition. De façon générale, leur quantité dépend de la quantité de polymère utilisée.

Après solubilisation ou dispersion du polymère dans les huiles à cycle carboné, il est possible d'ajouter tout autre huile ou solvant organique généralement utilisé dans les domaines considérés et compatible avec l'alkylcycloalcane, c'est-à-dire miscible avec l'alkylcycloalcane ou dispersable dans l'alkylcycloalcane.

On peut par exemple citer les huiles hydrocarbonées telles que l'huile de paraffine ou de vaseline, le polyisobutène hydrogéné, l'huile de vison, de tortue, de soja, le perhydrosqualène, l'huile d'amande douce, de calophyllum, de palme, de pépins de raisin, de sésame, de maïs, de parléam, d'arara, de colza, de tournesol, de coton, d'abricot, de ricin, d'avocat, de jojoba, d'olive ou de germes de céréales; des esters d'acide lanolique, d'acide oléique, d'acide laurique, d'acide stéarique; les esters d'acides ou d'alcools à chaîne grasse (ayant de 6 à 20 atomes de carbone), tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine; les acides gras supérieurs tels que l'acide myristique, l'acide palmitique, l'acide stéarique, l'acide béhénique, l'acide oléique, l'acide linoléique, l'acide linolénique ou l'acide isostéarique; les alcools gras supérieurs tels que l'alcool stéarylique, l'alcool oléique, l'alcool linoléique ou linolénique, l'alcool isostéarique ou l'octyl dodécanol; les huiles siliconées telles que les polydiméthylsiloxane (PDMS), éventuellement phénylées telles que les phényltriméthicones, les phényltriméthylsiloxydiphénylsiloxanes, les diphénylméthyl diméthyltrisiloxanes, les diphényldiméthicones, les phényl diméthicones, les polyméthylphénylsiloxanes, ou éventuellement substitués par des groupements aliphatiques et/ou aromatiques, éventuellement fluorés, ou par des groupements fonctionnels tels que des groupements hydroxyle, thiol et/ou amine; les polysiloxanes modifiés par des acides gras, des alcools gras ou des polyoxyalkylènes comme les alkyldiméthiconecopolyols, les silicones fluorées telles que les polydiméthylsiloxanes perfluoroalkylés et les polyméthylphénylsiloxanes perfluoroalkylés, les huiles perfluorées.

Avantageusement, on peut utiliser une ou plusieurs huiles volatiles à température ambiante. Ces huiles volatiles sont favorables à l'obtention d'un film à propriétés "sans transfert" total. Après évaporation de ces huiles, on obtient un dépôt filmogène souple, non collant sur la peau ou les muqueuses, suivant respectivement le mouvement de la peau ou des lèvres, sur lesquelles la composition est appliquée. Ces huiles volatiles facilitent, en outre, l'application de la composition sur la peau, les muqueuses, les phanères.

Ces huiles peuvent être des huiles hydrocarbonées, des huiles silicones comportant éventuellement des groupements alkyle ou alcoxy en bout de chaîne siliconée ou pendante.

Comme huile de silicone volatile utilisable dans l'invention, on peut citer les silicones linéaires ou cycliques ayant de 2 à 7 atomes de silicium, ces silicones comportant éventuellement des groupes alkyle ou alcoxy ayant de 1 à 10 atomes de carbone ainsi que les isoparaffines en C₈-C₁₆ et les huiles fluorées ou perfluorées volatiles. Ces huiles volatiles représentent notamment de 30 à 97,99% du poids total de la composition, et mieux de 30 à 75%.

Comme huile volatile utilisable dans l'invention, on peut citer notamment l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane ou les isoparaffines en C₈-C₁₆ telles que les 'ISOPARs', les PERMETYLs et notamment l'isododécane ou l'isohexadécane De préférence, on utilise des huiles volatiles hydrocarbonées comme les isoparaffines.

On peut également citer les solvants, seuls ou en mélange, choisis parmi les esters linéaires, ramifiés ou cycliques, ayant plus de 6 atomes de carbone, les éthers ayant plus de 6 atomes de carbone, les cétones ayant plus de 6 atomes de carbone et les alcools comme le décanol, de dodécanol et l'octadécanol.

Les huiles et/ou solvants complémentaires peuvent représenter de 0% à 40% du poids total de la composition, et de préférence de 1 à 30%.

La composition peut comprendre une matière colorante contenant un ou plusieurs composés pulvérulents et/ou un ou plusieurs colorants liposolubles, par exemple à raison de 0,01 à 70% du poids total de la composition. Les composés pulvérulents peuvent être choisis parmi les pigments et/ou les nacres et/ou les charges habituellement utilisés dans les compositions cosmétiques ou dermatologiques. Les composés pulvérulents peuvent représenter de 0,1 à 98% du poids total de la composition, et par exemple de 1 à 80%. Plus la quantité de composés pulvérulents diminue, plus les qualités de sans transfert et de confort augmentent. Aussi, de préférence ces composés pulvérulents représentent de 0,1 à 40%, et mieux de 1 à 30%. Le fait que les propriétés de sans transfert augmentent au fur et à mesure que la quantité de composés pulvérulents diminue, est tout à fait surprenant. En effet, jusqu'à ce jour, les propriétés de sans transfert des compositions de l'art antérieur augmentaient avec la quantité de composés pulvérulents. Inversement, leurs inconforts et leur sécheresse sur la peau ou muqueuses augmentaient.

Par ailleurs, la propriété de tenue prolongée et/ou de sans transfert augmente avec la quantité de polymère ou copolymère amorphe, soluble ou dispersable dans la phase grasse liquide. En pratique, le polymère ou copolymère amorphe peut représenter en matière active ou matière sèche jusqu'à 50% du poids total de la composition.

De préférence, il représente de 0,1 à 40 % et mieux de 1 à 30 % du poids total de la composition, et encore mieux de 5 à 30%.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon (Orgasol de chez Atochem), de poly-β-alamine et de polyéthylène, le Téflon, la lauroy-lysine, l'amidon, le nitrure de bore, les poudre de polymères de tétrafluoroéthylène, les microsphères creuses telles l'Expancel (Nobel Industrie), le polytrap (Dow Corning) et les microbilles de résine de silicone (Tospearls de Toshiba, par exemple), le carbonate de calcium précité, la carbonate et l'hydro-carbonate de magnésium, l'hydroxyapatite, les microsphères de silice creuses (SILICA BEADS DE MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zincs, de magnésium ou de lithium, le laurate de zinc, le myristate de magnésium.

Les charges utilisées, en particulier les charges organiques de nature polymère, peuvent être réticulées ou non et contenir à l'intérieur des particules un ou plusieurs actifs cosmétiques pouvant être libérés après application de la composition.

Les pigments et les charges peuvent être ou non enrobés superficiellement, en particulier traités en surface par des silicones, des acides aminés, des dérivés fluorés ou toute autre substance favorisant la dispersion et la compatibilité du pigment dans la composition.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine. Ils peuvent représenter de 0,01 à 20 % du poids de la composition et mieux de 0,1 à 6 %.

Le polymère ou copolymère amorphe de la composition de l'invention permet la formation d'un film sur la peau, les lèvres et/ou les cils, formant un réseau piégeant les matières colorantes et/ou les actifs. Selon la quantité relative de matières colorantes, utilisées par rapport à la quantité de polymère stabilisé, utilisée, il est possible d'obtenir un film plus ou moins brillant.

Comme actifs cosmétiques ou dermatologiques, utilisables dans la composition de l'invention, on peut citer les hydratants, les vitamines, les acides gras essentiels, les sphingolipides, les filtres ou bloqueurs solaires, les antioxydants, les antiacnés, les anti-inflammatoires, les agents bronzants (en l'absence de rayonnement UV), les agents dépigmentants, les agents mâtifiants et leurs mélanges. Ces actifs sont utilisés en quantité habituelle pour l'homme du métier et notamment à des concentrations de 0,001 à 20 % du poids total de la composition.

La composition selon l'invention peut, de plus, comprendre, selon le type d'application envisagée, les constituants classiquement utilisées dans les domaines considérés, qui sont présents en une quantité appropriée à la forme galénique souhaitée.

En particulier, elle peut comprendre, outre la phase grasse dans laquelle le polymère ou copolymère amorphe est présent, des phases grasses additionnelles qui peuvent être choisies parmi les cires, les gommes et/ou les corps gras pâteux, d'origine végétale, animale, minérale ou de synthèse, voire siliconé, et leurs mélanges.

Parmi les cires solides à température ambiante, susceptibles d'être présentes dans la composition selon l'invention, on peut citer les cires hydrocarbonées telles que la cire d'abeilles, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène, les cires obtenues par synthèse de Fischer-Tropsch, les huiles hydrogénées, les esters gras et les glycérides concrets à 25%C. On peut également utiliser des cires de silicone, parmi lesquelles on peut citer les alkyl, alcoxy et/ou esters de polyméthylsiloxane. Les cires peuvent se présenter sous forme de dispersions stables de particules colloïdales de cire telles qu'elles peuvent être préparées selon des méthodes connues, telles que celles de "Microemulsions Theory and Practice", L. M. Prince Ed., Academic press (1977, pages 21-32. Comme cire liquide à température ambiante, on peut citer l'huile de Jojoba.

Les cires peuvent être présentes à raison de 0-50 % en poids dans la composition et mieux de 1 à 30 %.

La composition peut comprendre, en outre, tout additif usuellement utilisé dans de telles compositions, tel que des épaississants, des parfums, des conservateurs, des tensioactifs, d'autres polymères liposolubles. Bien entendu l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

Les compositions selon l'invention peuvent en outre contenir des homopolymères et copolymères liposolubles et/ou dispersables dans la phase grasse, différents des poly(cyclooléfines) de l'invention.

Parmi ces homopolymères et copolymères, on peut citer des polyoléfines telles que le polyéthylène, le polybutène et le polydécène; des copolymères d'esters et/ou amides (méth)acryliques; des copolymères d'esters vinyliques, par exemple des copolymères éthylène/acétate de vinyle; des homopolymères ou copolymères vinyliques ou (méth)acryliques portant un groupement silicone tels que par exemple des copolymères greffés à squelette (méth)acrylique et greffons de silicone macromère; des copolymères à squelette ou séquences (méth)acrylique et à greffons ou séquences hydrocarbonés, par exemple polyisobutylène; des copolymères greffés ou séquencés à squelette ou séquence polyorganosiloxane et à greffons ou séquences (méth)acryliques et/ou vinyliques; des homopolymères ou copolymères fluorés ou perfluorés, par exemple des polyéthers perfluorés tels ceux commercialisés sous la désignation FOMBLINS®, des homo ou copolymères (méth)acryliques perfluorés, des homo ou copolymères vinyliques fluorés, des homo ou copolymères d'oléfines fluorés et des poly(éther vinyliques) fluorés.

Dans un mode de réalisation particulier de l'invention, les compositions selon l'invention peuvent être préparées de manière usuelle par l'homme du métier. Elle peuvent se présenter sous forme d'un produit visqueux éventuellement coulé et par exemple sous la forme d'un stick ou bâton, ou sous la forme de coupelle utilisable par contact direct ou à l'éponge. En particulier, elles trouvent une application en tant que fond de teint coulé, fard à joues ou à paupières coulé, rouge à lèvres, base ou baume de soin pour les lèvres, produit anti-cernes. Elles peuvent aussi se présenter sous forme d'une pâte souple, de viscosité dynamique à 25°C de l'ordre de 1 à 40 Pa.s ou encore de gel, de crème plus ou moins fluides. Elles peuvent alors constituer des fonds de teint ou des rouges à lèvres, des produits solaires ou de coloration de la peau, des mascaras, des eye-liners et des produits de maquillage du corps. Elles peuvent aussi se présenter sous forme de poudre libre ou compactée, le polymère servant alors de liant.

Les compositions de l'invention sont avantageusement anhydres et peuvent contenir moins de 5% d'eau par rapport au poids total de la composition. Elles peuvent alors se présenter notamment sous forme de gel huileux, de liquide huileux ou huile, de pâte ou de stick ou encore sous forme de dispersion vésiculaire contenant des lipides ioniques et/ou non ioniques. Ces formes galéniques sont préparées selon les méthodes usuelles des domaines considérés.

Les compositions selon l'invention peuvent également avantageusement se présenter sous forme d'émulsion eau-dans-huile, huile-dans-eau ou eau-dans-cire, dans lesquelles les copolymères d'oléfines selon l'invention sont utilisés pour remplacer tout ou partie des cires habituellement présentes dans ces émulsions. En particulier, la cire peut être constituée d'au moins un copolymère d'oléfines selon l'invention et d'au moins une huile, volatile ou non.

Ces compositions à applications topique peuvent constituer notamment une composition cosmétique, de traitement ou de soin pour le visage, pour le cou, pour les mains ou pour le corps (par exemple crème de soin, huile solaire, gel corporel), une composition de maquillage (par exemple gel de maquillage) ou une composition de bronzage artificiel ou de protection solaire.

### Exemples de formulations illustrant l'invention

### 1/ Exemples de comparaison décrivant les propriétés filmogènes

a) Comme exemple de poly(cyclooléfine), on utilise ici un copolymère amorphe éthylène/norbornène de T_{G} = + 85 °C, commercialisé par la société TICONA (USA) sous le nom commercial "TOPAS® 8007-S-04". Pour la réalisation du film et l'étude de ses caractéristiques, on réalise une solution à 5% de ce copolymère TOPAS® dans le cyclohexane par dissolution, on applique cette solution sur une plaque de Téflon® et on "tire" le film à l'aide d'un applicateur traditionnel pour obtenir un film d'épaisseur "humide" 300µm. On laisse sécher ce film par évaporation une journée à température ambiante.
On obtient alors un film transparent, lisse, brillant, suffisamment souple pour être décollé du support siliconé, non collant au toucher. Le film est alors coupé en plusieurs morceaux qui sont immergés soit dans l'eau, soit dans du "Teepol®" pur (comme exemple de tensio-actif concentré) : les morceaux du film sont alors observés à l'oeil qualitativement 15 minutes après immersion puis à des temps réguliers jusqu'à 72 heures d'immersion. On constate que les morceaux de film ne sont pas modifiés c'est-à-dire restent transparents, non collants au toucher, sans gonflement apparent, brillants, non cassants et de flexibilité comparable à celle avant immersion.
b) Comparaison à des films d'autres polymères commerciaux liposolubles.
On a réalisé les solutions du tableau ci-dessous et déterminé le caractère filmogène des solutions et la qualité des films éventuellement obtenus. Les résultats sont donnés dans le tableau ci-dessous.

| Polymère | Solvant | Film |
|---|---|---|
| Copolymère méthacrylate d'isobornyle, stéaryle et cétyle | isododécane | non filmogène |
| | | |
| Copolymère polyvinylpyrrolidone/hexadécène | cyclohexane | non filmogène |
| | | |
| Polyéthylène linéaire | isododécane ou cyclohexane | non filmogène |
| | | |
| Méthacrylate d'isobornyle, stéaryle et cétyle | isododécane | non filmogène |
| | | |
| Copolymère anhydride maléique/octadécylvinyléther | toluène | non filmogène |

Alors que le copolymère de cyclooléfine TOPAS® conduit à des films transparents, brillants, non collants, flexibles et non cassants qui sont très résistants à l'eau et aux tensio-actifs, aucun des autres copolymères testés ne conduit à des films.

### 2/ Exemples comparatifs décrivant les propriétés d'épaississement des huiles

Comme exemples de poly(cyclooléfines) amorphes, on utilise ici le même copolymère "TOPAS® 8007-S-04" commercialisé par TICONA et une autre copolyoléfine, de T_{G} = + 138°C, commercialisé par la société NIPPON ZEON sous le nom commercial "ZEONEX® 480".

On a réalisé des solutions à 5% en poids dans le dioctylcyclohexane du copolymère TOPAS® et du copolymère ZEONEX®, et à titre de comparaison, une solution à 10% en poids de copolymère triblocs KRATON® dans du benzoate d'alkyle C₁₂-C₁₅.

On a mesuré la viscosité réduite des différentes solutions :

| Solution | Viscosité réduite (centipoises) |
|---|---|
| TOPAS® 5% + dioctylcyclohexane | 6000 |
| | |
| ZEONEX® 5% + dioctylcyclohexane | 2000 |
| | |
| KRATON® 10% + C₁₂-C₁₅ alkylbenzoate | 1000 |

Ces résultats montrent que les copolymères de cyclooléfine selon l'invention ont des propriétés épaississantes de leur huile de dissolution très supérieures à celles de copolymères triblocs KRATON®.

### 3/ Exemple de produit solaire anhydre

- TOPAS® 8007-S-04* 5g
- TiO₂ nanométrique (MT 100T) 7g
- Isododécane 10g
- Dioctylcyclohexane qs 100g
*Température de transition vitreuse T_{g} + 85°C
Masse moléculaire (PM) de 5000 Dalton
Densité de 1,02

Le produit est obtenu en dissolvant le polymère dans le dioctylcyclohexane, puis en ajoutant l'isododécane et le TiO₂, sous agitation à température ambiante. Il présente des propriétés de résistance à l'eau, au sébum et au frottement remarquables.

## Revendications

1. Composition susceptible d'être appliquée sur les matières kératiniques, comprenant une phase grasse liquide, caractérisée par le fait qu'elle comprend une quantité efficace d'une poly(cyclooléfine) amorphe ou d'un mélange de poly(cyclooléfines) amorphes, la phase grasse liquide comprenant au moins une huile dont la structure chimique comporte au moins un noyau carboné.

2. Composition selon la revendication 1, caractérisée en ce que la ou les poly(cyclooléfines) amorphes ont une température de transition vitreuse allant de -20°C à +300°C, de préférence -10°C à +150°C.

3. Composition selon la revendication 1 ou 2, caractérisée en ce que la ou les poly(cyclooléfines) sont choisies parmi les homopolymères de cyclooléfines et les copolymères de cyclooléfines.

4. Composition selon la revendication 3, caractérisée en ce que les copolymères de cyclooléfines sont choisis parmi les copoly(oléfine/cyclooléfine).

5. Composition selon la revendication 4, caractérisée en ce que les copolymères de cyclooléfines comportent un taux de cyclooléfine égal ou supérieur à 10 moles %, de préférence égal ou supérieur à 20 moles %.

6. Composition selon l'une quelconque des revendications 1 à 5, caractérisée en ce que la poly(cyclooléfine) amorphe ou le mélange de poly(cyclooléfines) amorphes représente au moins 1 % en poids, par rapport au poids total de la composition.

7. Composition selon l'une quelconque des revendications précédentes, caractérisée par le fait que la poly(cyclooléfine) amorphe ou le mélange de poly(cyclooléfines) amorphes est filmifiable.

8. Composition selon l'une quelconque des revendications 1 à 7, caractérisée par le fait qu'elle se présente sous la forme d'un produit épaissi comprenant une phase grasse liquide cosmétique et au moins une cire solide.

9. Composition selon la revendication 8, caractérisée par le fait que la phase grasse liquide comprend des huiles volatiles.

10. Composition selon l'une quelconque des revendications précédentes, caractérisée par le fait qu'elle comprend, en outre, au moins un actif choisi parmi les actifs cosmétiques et dermatologiques.

11. Composition selon l'une quelconque des revendications précédentes, caractérisée par le fait qu'elle comprend, de plus, au moins une matière colorante.

12. Composition selon l'une quelconque des revendications précédentes, caractérisée par le fait que la poly(cyclooléfine) amorphe résulte de la polymérisation ou copolymérisation d'au moins une cyclooléfine choisie parmi le cyclobutène, le cyclohexène, le cyclooctène, le norbornène, le 5-méthylnorbornène, le 5-éthylnorbornène, le 5,6-diméthylnorbornène, le 5,5,6-triméthylnorbornène, le 5-éthylidène norbornène, le 5-phénylnorbornène, le 5-benzylnorbornène, le 5-vinylnorbornène, le 1,4,5,8-diméthano-1,2,3,4,4a,5,8a-octahydronaphtalène, le dicyclopentadiène et leurs mélanges.

13. Composition selon l'une quelconque des revendications 1 à 11, caractérisé en ce que la poly(cyclooléfine) amorphe est choisi parmi les poly(norbornènes), les poly(cyclobutènes), les polycyclohexènes, les poly(éthylène-co-norbornènes), les poly(éthylène-co-cyclobutènes), les poly(éthylène-co-cyclohexènes) et les terpolymères éthylène /propylène/éthylidène-norbornènes.

14. Composition selon la revendication 13, caractérisée en ce que les poly(éthylène-co-norbomènes) ont une teneur en norbornène égale ou supérieure à 20 moles %.

15. Composition selon l'une quelconque des revendications 1 à 14, caractérisée en ce que les poly(cyclooléfines) amorphes sont obtenues par synthèse classique, métathèse ou métallocène.

16. Composition selon l'une quelconque des revendications précédentes, dans laquelle la phase grasse liquide comprend des huiles d'origine minérale, animale, végétale ou synthétique, carbonées, hydrocarbonées, fluorées et/ou siliconées, seules ou en mélange.

17. Composition selon l'une quelconque des revendications précédentes dans laquelle la phase grasse liquide contient au moins un (alkyl)cycloalcane.

18. Composition selon l'une quelconque des revendications précédentes, dans laquelle la phase grasse liquide contient au moins une huile choisie parmi l'huile de paraffine ou de vaseline, l'huile de vison, de tortue, de soja, le perhydrosqualène, l'huile d'amande douce, de calophyllum, de palme, de parléam, de pépins de raisin, de sésame, de maïs, de colza, de tournesol, de coton, d'abricot, de ricin d'avocat, de jojoba, d'olive ou de germes de céréales ; des esters d'acide lanolique, d'acide oléique, d'acide laurique, d'acide stéarique ; les esters gras tels quele myristate d'isopropyle, le palmitate d'isopropyle, le stéarate de butyle, le laureate d'hexyle, l'adipate de diisopropyle, l'isonate d'isononyle, le palmitate de 2-éthyl-hexyle, le laurate de 2-hexyl-décyle, le palmitate de 2-octyl-décyle, le myristate ou le lactate de 2-octyldodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les PDMS éventuellement phénylés tels que les phényltriméthicones, les phényltriméthylsiloxydiphénylsiloxanes, les diphénylméthyldiméthyltrisiloxanes, les diphényldiméthicones, les phényldiméthicones, les polyméthylphénylsiloxanes ou éventuellement substitutés par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées ; les huiles volatiles telles que l'octaméthylcyclotétrasiloxane, le décaméthylcyclopentasiloxane, l'hexadéméthycyclohexasiloxane, l'heptaméthylhexyltrisiloxane, l'heptaméthyloctyltrisiloxane ou les isoparaffines en C₈-C₁₆, et notamment l'isododécane et l'isohexadécane.

19. Composition selon l'une quelconques des revendications précédentes, dans laquelle la phase grasse liquide contient au moins une huile hydrocarbonée volatile à température ambiante.

20. Composition selon l'une des revendications précédentes, comprenant, en outre, au moins une phase grasse additionnelle choisie parmi les cires, les gommes et/ou les corps gras pâteux, d'origine végétale, animale, minérale, de synthèse, ou siliconé, et leurs mélanges.

21. Composition selon l'une quelconque des revendications 11 à 20, caractérisée en ce que la matière colorante comprend au moins un composé pulvérulent choisi parmi les charges, les pigments, les nacres et leurs mélanges.

22. Composition selon la revendication 21, caractérisée en ce que le composé pulvérulent représente 0,1 à 98% du poids total de la composition.

23. Composition selon la revendication 21, caractérisée en ce que le composé pulvérulent représente de 1 à 30% du poids total de la composition.

24. Composition selon l'une des revendications précédentes, caractérisée en ce que la poly(cyclooléfine) amorphe ou leur mélange représente (en matière sèche) jusqu'à 50% du poids total de la composition.

25. Composition selon l'une des revendications 1 à 23, caractérisée en ce que la poly(cyclooléfine) amorphe ou leur mélange représente (en matière sèche) de 5 à 30% du poids total de la composition.

26. Composition selon l'une quelconque des revendications précédentes, caractérisée en ce que la phase grasse liquide contient au moins une huile choisie parmi les isoparaffines en C₈-C₁₆.

27. Composition selon l'une quelconque des revendications précédentes, se présentant sous forme d'un stick ou bâton; sous la forme d'une pâte souple, de viscosité dynamique à 25°C de l'ordre de 1 à 40 Pa.s ; sous forme de coupelle ; de gel huileux ; de liquide huileux ; de dispersion vésiculaire contenant des lipides ioniques et/ou non ioniques, d'une émulsion eau-dans-huile, huile-dans-eau ou eau-dans-cire, ladite cire pouvant être constituée par un mélange d'au moins une poly(cyclooléfine) amorphe ou un mélange de celles-ci avec au moins une huile dont la structure chimique comporte au moins un cycle carboné.

28. Composition selon l'une quelconque des revendications 1 à 26, se présentant sous forme anhydre.

29. Composition selon l'une des revendications précédentes, se présentant sous la forme d'un produit de soin et/ou de maquillage de la peau et/ou des lèvres et/ou des cils.

30. Composition selon l'une des revendications précédentes, se présentant sous la forme d'un fond de teint coulé, d'un fard à joues ou à paupières coulé, d'un rouge à lèvres, d'une base ou baume de soin pour les lèvres, d'un produit anti-cernes, d'un mascara, d'un eye-liner ou d'un maquillage du corps.

31. Utilisation dans une composition non thérapeutique ou pour la fabrication d'une composition dermatologique contenant au moins une phase grasse liquide, d'une quantité efficace d'une poly(cyclooléfine) amorphe ou d'un mélange de poly(cyclooléfines) amorphes, soluble ou dispersable dans ladite phase grasse liquide, pour épaissir et/ou gélifier ladite phase grasse liquide, la phase grasse liquide comprenant au moins une huile dont la structure chimique comporte au moins un cycle carboné.

32. Utilisation dans ou pour la fabrication d'une composition susceptible d'être appliquée sur les matières kératiniques, comprenant une phase grasse liquide et au moins un ingrédient choisi parmi les actifs cosmétiques ou dermatologiques, les matières colorantes et leurs mélanges, d'au moins une poly(cyclooléfine) amorphe ou un mélange de poly(cyclooléfines) amorphes soluble ou dispersable dans ladite phase grasse liquide, pour augmenter la tenue et/ou pou diminuer, voire supprimer, le transfert du film de composition déposé sur la peau et/ou les lèvres et/ou les phanères, la phase grasse liquide comprenant au moins une huile dont la structure chimique comporte au moins un cycle carboné.

33. Utilisation pour réaliser un film résistant à l'eau et/ou au sébum, non collant et/ou confortable à porter d'une poly(cyclooléfine) amorphe ou d'un mélange de poly(cyclooléfines) amorphes dissous ou dispersé dans une phase grasse liquide comprenant au moins une huile dont la structure chimique comporte au moins un cycle carboné.

34. Utilisation dans ou pour la fabrication d'une composition cosmétique susceptible d'être appliquée sur les matières kératiniques, d'une poly(cyclooléfine) amorphe ou d'un mélange de poly(cyclooléfines) amorphes dissous ou dispersé dans une phase grasse liquide comprenant au moins une huile dont la structure chimique comporte au moins un cycle carboné.

35. Utilisation selon la revendication 31 ou 34, caractérisée en ce que la poly(cyclooléfine) amorphe est choisie parmi les poly (norbornènes), les poly(cyclobutènes), les poly(cyclohexènes), les poly(éthylène-co-norbomènes), les poly(éthylène-co-cyclobutènes), les poly(éthylène-co-cyclohexènes) et les poly(éthylène/propylène/éthylidène norbornène.

36. Utilisation selon l'une quelconque des revendications 31 à 35, caractérisée en ce que la poly(cyclooléfine) amorphe est obtenue par métathèse ou par synthèse métallocène.
